# EUROPEAN PATENT APPLICATION

(11) **EP 4 643 794 A1**
(43) Date of publication of application: **05.11.2025**
(21) Application number: 23909243.0
(22) Date of filing: 25.08.2023
(51) Int. Cl.: A61B 17/128, A61B 17/122

(54) **SPLIT-TYPE LEFT ATRIAL APPENDAGE CLOSURE CLAMP SYSTEM**

(30) Priority: 28.12.2022 CN 202211695298
(71) Applicant: Hangzhou Sunstone Technology Co., Ltd., Hangzhou, Zhejiang 310018 (CN)
(72) Inventor: GUO, Haiping, Hangzhou, Zhejiang 310018 (CN); SHI, Lei, Hangzhou, Zhejiang 310018 (CN); ZHANG, Wenjun, Hangzhou, Zhejiang 310018 (CN); WENG, Yaxue, Hangzhou, Zhejiang 310018 (CN); CHEN, Xiaorong, Hangzhou, Zhejiang 310018 (CN); TIAN, Zhuang, Hangzhou, Zhejiang 310018 (CN); LI, Zhonghui, Hangzhou, Zhejiang 310018 (CN); BAN, Zhisha, Hangzhou, Zhejiang 310018 (CN)
(74) Representative: Longoni, Alessandra
(86) International application number: PCT/CN2023/115014
(87) International publication number: WO 2024/139303

(57) **Abstract**

The present invention discloses a split-type left atrial appendage closure clip system, which includes a clip head and a clip applier. The clip applier includes a fixed handle, a clamping handle, a deflection adjustment sleeve, a clamping transmission member, a deflection transmission member, and a connecting tube. The clamping handle is slidably connected to the fixed handle. The deflection adjustment sleeve is threadedly connected to the fixed handle. The clamping transmission member is connected between the clamping handle and the clip head. The deflection transmission member is connected between the deflection adjustment sleeve and the clip head. The connecting tube is connected between the clip head and the fixed handle. The clamping transmission member and the deflection transmission member are threaded through the connecting tube. A clip head deflection mechanism driven by the deflection transmission member is provided between the clip head and the clip applier. By adopting the present invention, the closure method of the left atrial appendage can be changed from the ligation and suture method to the clip compression closure method, which is convenient and fast to operate, can avoid bleeding, and has complete closure, thus improving the convenience, safety and effectiveness of the left atrial appendage closure device

## Description

### Technical Field

The present invention relates to a cardiac surgical instrument, and more specifically, to A split-type left atrial appendage closure clip system.

### Background Technology

Atrial fibrillation is one of the important causes of stroke. Current research shows that most of the thrombi causing atrial fibrillation-related stroke originate from the left atrial appendage. Due to its special anatomical and functional characteristics, the left atrial appendage has become a research hotspot in atrial fibrillation-related stroke. In cardiac surgery, most left atrial appendage management is performed simultaneously with conventional thoracotomy, and some patients also have left atrial appendage management during surgery assisted by minimally invasive thoracoscopy. If a patient with simple atrial fibrillation needs to undergo surgical radiofrequency ablation of atrial fibrillation, currently, most adopt thoracoscopy assisted radiofrequency ablation of atrial fibrillation with simultaneous left atrial appendage management. Whether the left atrial appendage is managed via thoracotomy or thoracoscopy, the traditional methods are to use suture ligation, resection and suture, or use a cutting stapler to manage the left atrial appendage, but there are certain operational difficulties and complications are likely to occur. The advantages of suturing after resecting the left atrial appendage are thorough removal and a low rate of residual cavity, while the disadvantages are cumbersome operation and a high probability of bleeding. The advantage of the method of ligating the left atrial appendage is simplicity and rapidity, but there is indeed a possibility that the left atrial appendage cannot be completely sealed. If this probability is to be reduced, the suture ligation force needs to be increased, which may lead to the possibility of cutting the root of the left atrial appendage and increase the surgical risk. The root of the left atrial appendage is a flat-shaped structure, and clamping the left atrial appendage is more in line with its anatomical structure, making it easier to close the root of the left atrial appendage and avoiding the potential risks of residual cavity and incomplete ligation. For this reason, the clamping method can be considered, which avoids the disadvantages of both the resection-suture and ligation methods and has the advantages of both. The invention with the publication number CN114869381A discloses an atrial appendage clip, including a ligation wire and a guide rod. The ligation wire encloses to form a ligation ring, which can ligate and close the left atrial appendage, and the guide rod can guide the ligation ring to the surgical position. This invention can reduce the surgical wound and reduce the obstruction of the surgical field of view by the atrial appendage clip. However, this invention essentially belongs to the ligation method, so it is still difficult to overcome the inherent defects of the ligation method.

### Invention Content

The existing closure methods used in left atrial appendage surgery have problems such as incomplete ligation, easy bleeding, and high surgical risks. To overcome these defects, the present invention provides a split-type left atrial appendage closure clip system, which can conveniently and safely achieve the closure of the left atrial appendage in a closed manner.

The technical solution of the present invention is: A split-type left atrial appendage closure clip system, characterized in that it comprises a clip head and a clip applier. The clip applier includes a fixed handle, a clamping handle, a deflection adjustment sleeve, a clamping transmission member, a deflection transmission member, and a connecting tube. The clamping handle is slidably connected to the fixed handle. The deflection adjustment sleeve is threadedly connected to the fixed handle. The clamping transmission member is connected between the clamping handle and the clip head. The deflection transmission member is connected between the deflection adjustment sleeve and the clip head. The connecting tube is connected between the clip head and the fixed handle. The clamping transmission member and the deflection transmission member are threaded through the connecting tube. A clip head deflection mechanism driven by the deflection transmission member is provided between the clip head and the clip applier. When using the present invention, a clip body is loaded on the clip head, the clip head enters the body through a small incision channel, covers the left atrial appendage, and works under the control of the external clip applier. Operate the clamping handle, and the clip body is closed through the clamping transmission member to achieve the closure of the left atrial appendage. The clip head deflection mechanism drives the clip head to deflect under the control of the deflection adjustment sleeve and the deflection transmission member, adjusts the relative angle with the connecting tube, so that the clip head can cover the left atrial appendage at the optimal angle, avoids blocking the line of sight of the surgical operator, and enables the surgical operator to obtain the optimal field of view and closure angle. The minimally invasive left atrial appendage clipping method under thoracoscopy based on the split-type left atrial appendage closure clip system intervenes in the left atrial appendage outside the heart, has a high closure rate, simple operation, high safety, small trauma, quick recovery, is easier to operate, and has few complications.

Preferably, the fixed handle comprises a handle rod. The clamping handle is slidably sleeved on the handle rod. A pull-head slot is provided on the circumferential surface of the handle rod along the axial direction of the handle rod. The end of the clamping transmission member is fixedly connected to the clamping handle. When a force is applied to the clamping handle to make the clamping handle slide on the handle rod, the clamping handle also drives the end of the clamping transmission member to slide, pulls the clamping transmission member and transmits the pulling force to the clip head, and controls the clip body to complete the closing action.

Preferably, the clamping transmission member is connected to the clamping handle through a pin rod connected to the clamping handle. The connection between the clamping handle and the clamping transmission member is realized through the pin rod, and the structure is simple and convenient to implement.

Preferably, a step is provided on the inner wall of the central hole of the deflection adjustment sleeve. A pull head is provided at the rear end of the deflection transmission member. A pair of pull-head pins are radially fixed on the pull head. The pull-head pins are threaded through the pull-head slot and extend outside the pull-head slot. The part of the pull-head pin exposed outside the pull-head slot is clamped on the step of the inner wall of the central hole of the deflection adjustment sleeve. When deflecting the clip head, only the deflection adjustment sleeve needs to be rotated. The relative rotation between the deflection adjustment sleeve and the fixed handle is converted into axial movement through threads. The steps on the inner wall of the central hole of the deflection adjustment sleeve push the pull head of the deflection transmission member to move, so that the deflection transmission member generates axial pulling force, which is transmitted to the clip head to provide power for the deflection of the clip head.

Preferably, the clamping transmission member is a cable, which is threaded through the deflection transmission member, and the deflection transmission member is threaded inside the handle rod. The clamping transmission member and the deflection transmission member pass through the centers of the handle rod and the connecting tube, and the transmission paths are hidden, which is beneficial for the present invention to maintain a compact structure, a simple appearance, and reliable operation.

Preferably, the clip head comprises a movable clamp arm and a clip head frame. A first clamping body is detachably connected to the movable clamp arm. A second clamping body is detachably connected to the frame of the clip head frame opposite to the movable clamp arm. A clamping and locking structure is provided between the first clamping body and the second clamping body. The movable clamp arm is slidably connected inside the clip head frame and connected to the clamping transmission member. A rubber band for resetting the movable clamp arm is provided between the movable clamp arm and the clip head frame. A clip body is installed on each of the movable clamp arm and the frame of the clip head frame opposite to the movable clamp arm. The force acting on the clamping handle is transmitted to the movable clamp arm through the clamping transmission member, pulling the movable clamp arm to slide against the reset force of the rubber band. The first clip body gradually approaches the second clip body. When the first clip body and the second clip body are brought together, and the two clip bodies clamp the left atrial appendage through the clamping and locking structure to close the clip system, the first clip body and the second clip body can be separated from the movable clamp arm and the frame of the clip head frame through subsequent operations, and the clip bodies can also be removed from the human body, so that the use is faster, and the retention time of the clip bodies in the human body is reduced.

Preferably, the clip head deflection mechanism comprises a connecting lug and a deflection push rod. The connecting lug is provided on the clip head frame. The connecting lug is hingedly connected to the end of the connecting tube. One end of the deflection push rod is hingedly connected to the connecting lug, and the other end of the deflection push rod is hingedly connected to the deflection transmission member. After the deflection transmission member obtains the driving force from the deflection adjustment sleeve, it drives the connecting lug to rotate around the hinge point between the connecting tube and the connecting lug, thereby realizing the deflection of the clip head.

Preferably, a slider guide slot is provided on the frame of the clip head frame. A release slider is slidably connected inside the slider guide slot. A C-shaped elastic piece is sleeved on the frame of the clip head frame. The second clamping body abuts against the inner side of the frame of the clip head frame under the constraint of the C-shaped elastic piece. An inclined surface in contact with the C-shaped elastic piece is provided on the back of the release slider. An unlocking cable is connected to the release slider. The unlocking cable is threaded through the connecting tube and connected to a release handle. The release handle is hingedly connected to a release handle ring, and the release handle ring is fixed on the fixed handle. After the first clip body and the second clip body clamp the left atrial appendage to close the clip system, the first clip body and the second clip body are connected together through a clamping and locking structure. At this time, the first is to loosen the clamping handle, and the pulling force of the clamping transmission member disappears, and the rubber band drives the movable clamp arm to reset. Since the second clip body is constrained on the clip head frame by the elastic piece and cannot move along with the first clip body, and the binding force between the first clip body and the second clip body is greater than the binding force between the first clip body and the movable clamp arm. Therefore, the first clip body is carried away from the movable clamp arm by the second clip body; then press the release handle to drive the unlocking cable, and the unlocking cable pulls the release slider to slide in the slider guide slot. The part where the inclined surface on the back of the release slider contacts the C-shaped elastic piece gradually transfers from the low point to the high point of the inclined surface, and the pressure between the inclined surface and the C-shaped elastic piece becomes increasingly large until the C-shaped elastic piece is opened up to make it unable to restrict the second clip body in the slider guide slot. The second clip body then releases the constraint with the release slider and detaches from the release slider. In this way, both the first clip body and the second clip body are completely detached from the clip head.

Preferably, the first clamping body and the second clamping body are made of POM. When necessary, the first clip body and the second clip body need to be permanently placed in the human body. POM has stable properties, is relatively friendly to the human body environment, will not interfere with imaging examinations, and will not cause human rejection reactions.

Preferably, the connecting tube comprises two half-section tubes, and the two half-section tubes are joined together. The connecting tube is formed by joining two half-section tubes, which is convenient for arranging the clamping transmission member and the deflection transmission member inside the connecting tube.

The beneficial effects of the present invention are:
Improving the convenience, safety and effectiveness of the left atrial appendage closure device. By adopting the present invention, the closure method of the left atrial appendage can be changed from the ligation and suture method to the clip compression closure method, which is convenient and fast to operate, can avoid bleeding, and has complete closure.

### Description of the Drawings

Picture 1 is a structural schematic diagram of the present invention;
Picture 2 is a structural schematic diagram of the present invention after removing the connecting tube;
Picture 3 is a schematic diagram of the transmission structure between the clip head and the clip applier in the present invention;
Picture 4 is a structural schematic diagram of the fixed handle in the present invention;
Picture 5 is a schematic diagram of the transmission structure between the clip head deflection mechanism and the deflection adjustment sleeve in the present invention;
Picture 6 is a schematic diagram of the disassembled structure of the clip applier in the present invention;
Picture 7 is a schematic diagram of the matching structure between the pull head of the deflection transmission member and the deflection adjustment sleeve in the present invention;
Picture 8 is a schematic diagram of the separated state of the half-section tube and the deflection transmission member in the present invention;
Picture 9 is a schematic diagram of the transmission structure between the clip head and the clip applier from another viewing angle in the present invention;
Picture 10 is a schematic diagram of the disassembled structure of the clip head in the present invention;
Picture 11 is a schematic diagram of the matching relationship among the release slider, the C-shaped elastic piece and the second clip body in the present invention.

In the pictures: 1- movable clamp arm, 2- release slider, 201- inclined surface, 202- unlocking cable, 3- fixed handle, 301- handle rod, 302- handle grip, 303- pull-head slot, 304- transition slot, 4- clamping handle, 401- pin rod, 5- deflection adjustment sleeve, 6- clamping transmission member, 7- deflection transmission member, 701- pull head, 702- tube cavity, 703- return spring, 704- pull-head pin, 8-connecting tube, 801- half-section tube, 802- front end limiting block, 9- clip head frame, 901- connecting lug, 902- deflection push rod, 903- slider guide slot, 904- C-shaped elastic piece, 10- release handle, 11- release handle ring, 12- first clip body, 13- second clip body, 1201- elastic buckle, 1301- toothed slot, 14- rubber band.

### Specific Implementation Methods

The present invention will be further described below in combination with the specific embodiments of the accompanying drawings.

### Embodiment 1:

As shown in Picture 1 to 11, a split-type left atrial appendage closure clip system includes a clip head and a clip applier. The clip applier includes a fixed handle 3, a clamping handle 4, a deflection adjustment sleeve 5, a clamping transmission member 6, a deflection transmission member 7, and a connecting tube 8. The clamping handle 4 is slidably connected to the fixed handle 3. The deflection adjustment sleeve 5 is threadedly connected to the fixed handle 3. The clamping transmission member 6 is connected between the clamping handle 4 and the clip head. The deflection transmission member 7 is connected between the deflection adjustment sleeve 5 and the clip head. The connecting tube 8 is connected between the clip head and the fixed handle 3. The clamping transmission member 6 and the deflection transmission member 7 are threaded through the connecting tube 8. A clip head deflection mechanism driven by the deflection transmission member 7 is provided between the clip head and the clip applier. The fixed handle 3 includes a hollow handle rod 301 and a handle grip 302. The clamping handle 4 is slidably sleeved on the handle rod 301. A pull-head slot 303 is provided on the circumferential surface of the threaded section of the handle rod 301 along the axial direction of the handle rod 301. A transition slot 304 radially penetrating the handle rod 301 is also arranged on the handle rod 301 along the axial direction. The end of the clamping transmission member 6 is fixedly connected to the clamping handle 4, and the connecting tube 8 is interference-fitted and inserted into the front port of the handle rod 301. The clamping transmission member 6 is connected to the clamping handle 4 through a pin rod 401. The pin rod 401 and the clamping handle 4 are integrally formed and penetrate the transition slot 304, and the end of the clamping transmission member 6 is wound around the pin rod 401. A step is provided on the inner wall of the central hole of the deflection adjustment sleeve 5. A pull head 701 is provided at the rear end of the deflection transmission member 7. A pair of pull-head pins 704 are radially fixed on the pull head 701. The pull-head pins 704 are threaded through the pull-head slot 303 and extend outside the pull-head slot 303. The part of the pull-head pin 704 exposed outside the pull-head slot 303 is clamped on the step of the inner wall of the central hole of the deflection adjustment sleeve 5. The clamping transmission member 6 is a steel cable, and the deflection transmission member 7 is a semi-open tube fitting with a bow-shaped cross-section and has a tube cavity 702. The cable is penetrated in the tube cavity 702 of the deflection transmission member 7, and the deflection transmission member 7 is penetrated in the handle rod 301. The clip head includes a movable clamp arm 1 and a clip head frame 9. A first clip body embedding slot is arranged on the inner side of the movable clamp arm 1, and a first clip body 12 is fittingly embedded in the first clip body embedding slot, so that the first clip body 12 is detachably connected to the movable clamp arm 1. A second clip body 13 is detachably connected to the upper frame of the clip head frame 9. A clamping and locking structure is arranged between the first clip body 12 and the second clip body 13. The clamping and locking structure includes elastic buckle 1201 located at both ends of the first clip body 12 and toothed slots 1301 located at both ends of the second clip body 13 and capable of matching with the elastic buckle 1201. The movable clamp arm 1 is slidably connected in the clip head frame 9 and connected to the clamping transmission member 6. A rubber band 14 for resetting the movable clamp arm 1 is arranged between the movable clamp arm 1 and the clip head frame 9. The rubber band 14 is fixed on the lower frame of the clip head frame 9. Under the action of the rubber band 14 in a normal state, the movable clamp arm 1 is close to the lower frame. T-shaped guide slots are arranged on the inner walls of both sides of the clip head frame 9. Both ends of the movable clamp arm 1 are fittingly and slidably clamped with the T-shaped guide slots, and the release slider 2 is arranged on one frame of the clip head frame 9. The clip head deflection mechanism includes a connecting lug 901 and a deflection push rod 902. The connecting lug 901 is arranged on the clip head frame 9 and is integrally formed with the clip head frame 9. A U-shaped connecting seat is arranged at the end of the connecting tube 8. The connecting lug 901 is embedded in the U-shaped connecting seat and is hinged to the end of the connecting pipe 8 through a first rotating shaft. One end of the deflection push rod 902 is hinged to the connecting lug 901 through a second rotating shaft, and the other end of the deflection push rod 902 is hinged to the deflection transmission member 7. A slider guide slot 903 is arranged on the outer side of the upper frame of the clip head frame 9, and a release slider 2 is slidably connected in the slider guide slot 903. A second clip body embedding slot is arranged on the inner side of the upper frame of the clip head frame 9. A C-shaped elastic piece 904 is sleeved on the upper frame of the clip head frame 9. The second clip body 13 is embedded in the second clip body embedding groove of the frame of the clip head frame 9 under the constraint of the opening side of the C-shaped elastic piece 904. The C-shaped elastic piece 904 is clamped in the elastic piece clamping slot on the frame, and the opening of the C-shaped elastic piece 904 is located on the inner side of the frame. In a normal state, the C-shaped elastic piece 904 contracts under its own elastic force, and the opening gap is reduced, which is sufficient to hang the second clip body 13, so as to realize the detachable connection of the second clip body 13 on the upper frame of the clip head frame 9. The movable clamp arm 1, the release slider 2, and the clip head frame 9 are all made of plastic. An inclined surface 201 abutting against the C-shaped elastic piece 904 is arranged on the back of the release slider 2. An unlocking cable 202 is connected to the release slider 2, the unlocking cable 202 is penetrated in the connecting tube 8 and connected to a release handle 10, the release handle 10 is hinged to a release handle ring 11, and the release handle ring 11 is fixedly sleeved on the handle rod 301 of the fixed handle 3. The connecting tube 8 includes two half-section tubes 801, and the two half-section pipes 801 are joined together. A front end limiting block 802 is arranged in one of the half-section tubes 801, the front end limiting block 802 is embedded in the tube cavity 702, a return spring 703 is embedded in the front end of the tube cavity 702, one end of the return spring 703 abuts against the front end wall of the tube cavity 702, and the other end abuts against the front end limiting block 802.

When using the present invention, the first clip body 12 and the second clip body 13 are loaded on the clip head. The first clip body 12 and the second clip body 13 are made of POM, a polymer implantable material, which has good biocompatibility. The clip head enters the body through a small incision channel, and is displayed by a thoracoscope to cover the left atrial appendage. Then, the relative angle between the clip head and the connecting tube 8 is adjusted through the clip head deflection mechanism, so that the clip head can be inserted into the left atrial appendage at the optimal angle, avoiding blocking the line of sight of the surgical operator and enabling the surgical operator to obtain the optimal field of view. When deflecting the clip head, only the deflection adjustment sleeve 5 needs to be rotated. The relative rotation between the deflection adjustment sleeve 5 and the fixed handle 3 is converted into axial movement through threads. The step on the inner wall of the central hole of the deflection adjustment sleeve 5 pushes the pull head 701 of the deflection transmission member 7 to move, so that the deflection transmission member 7 generates an axial pulling force, which is transmitted to the clip head to provide power for the deflection of the clip head. Hold the handle grip 302 and the clamping handle 4 with the hand, and grip and press the clamping handle 4. The clamping handle 4 carries the end of the clamping transmission member 6 to approach the handle grip 302. The clamping transmission member 6 pulls the movable clamp arm 1 to slide against the reset force of the rubber band 14 and gradually approaches the upper frame of the clip head frame 9. When the first clip body 12 and the second clip body 13 are brought together, the elastic buckle 1201 are clamped into the toothed slots 1301, and the first clip body 12 and the second clip body 13 are closed to achieve the closure of the left atrial appendage, and the two clip bodies are connected together through the clamping and locking structure. After the first clip body 12 and the second clip body 13 clamp the left atrial appendage to close the clip system, at this time, the first is to loosen the clamping handle 4, and the pulling force of the clamping transmission member 6 disappears, and the rubber band 14 drives the movable clamp arm 1 to reset. Since the second clip body 13 is constrained on the clip head frame 9 by the C-shaped elastic piece 904 and cannot move along with the first clip body 12, and the binding force between the first clip body 12 and the second clip body 13 is greater than the binding force between the first clip body 12 and the movable clamp arm. Therefore, the first clip body 12 is carried away from the movable clamp arm 1 by the second clip body 13; then press the release handle 10 to drive the unlocking cable 202, and the unlocking cable 202 pulls the release slider 2 to slide in the slider guide slot 903. The part where the inclined surface 201 on the back of the release slider 2 contacts the C-shaped elastic piece 904 gradually transfers from the low point to the high point of the inclined surface 201, and the pressure between the inclined surface 201 and the C-shaped elastic piece 904 becomes increasingly large until the C-shaped elastic piece 904 is opened up to make it unable to restrict the second clip body 13 in the slider guide slot. The second clip body 13 then releases the constraint with the release slider 2 and detaches from the release slider 2. In this way, both the first clip body 12 and the second clip body 13 are completely detached from the clip head.

### Embodiment 2:

The clamping and locking structure includes toothed slots located at both ends of the first clip body 12 and elastic buckle located at both ends of the second clip body 13 and capable of matching with the toothed slots. The rest is the same as Embodiment 1.

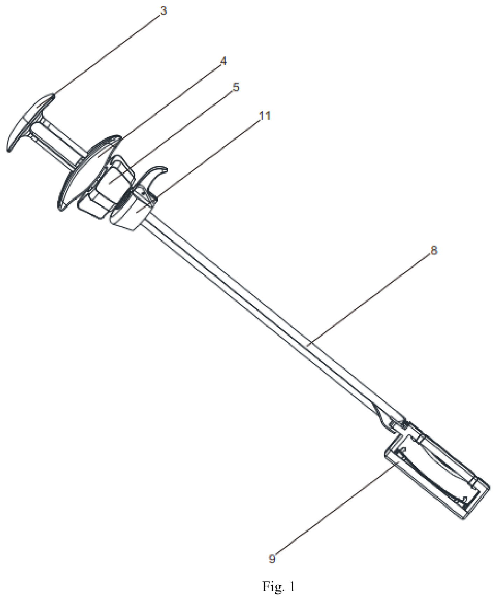

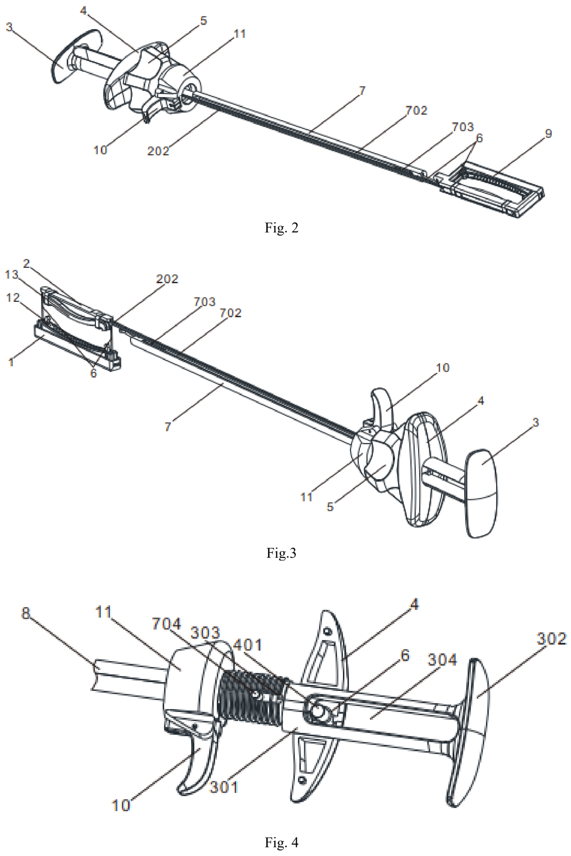

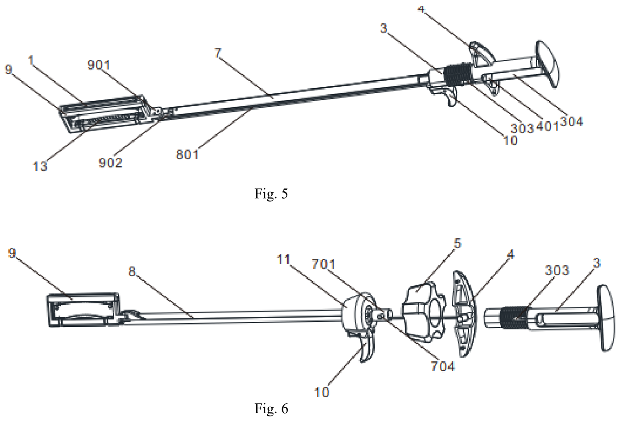

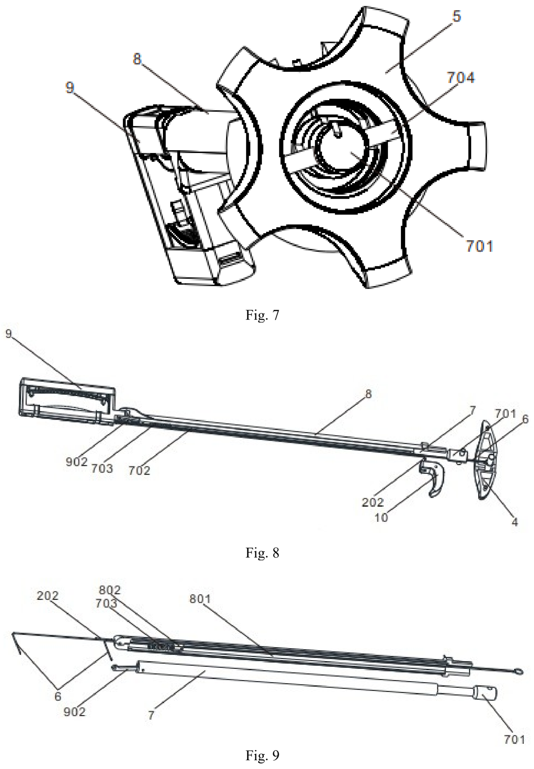

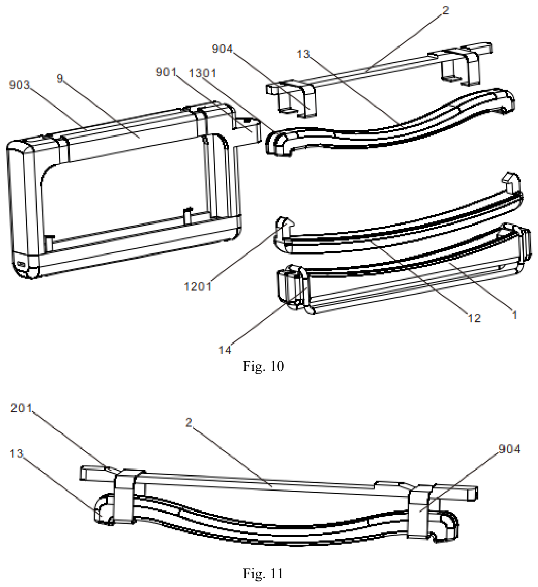

## Claims

1. A split-type left atrial appendage closure clip system, **characterized in that** it comprises a clip head and a clip applier. The clip applier includes a fixed handle (3), a clamping handle (4), a deflection adjustment sleeve (5), a clamping transmission member (6), a deflection transmission member (7), and a connecting tube (8). The clamping handle (4) is slidably connected to the fixed handle (3). The deflection adjustment sleeve (5) is threadedly connected to the fixed handle (3). The clamping transmission member (6) is connected between the clamping handle (4) and the clip head. The deflection transmission member (7) is connected between the deflection adjustment sleeve (5) and the clip head. The connecting tube (8) is connected between the clip head and the fixed handle (3). The clamping transmission member (6) and the deflection transmission member (7) are threaded through the connecting tube (8). A clip head deflection mechanism driven by the deflection transmission member (7) is provided between the clip head and the clip applier.

2. The split-type left atrial appendage closure clip system according to Claim 1, **characterized in that** the fixed handle (3) comprises a handle rod (301). The clamping handle (4) is slidably sleeved on the handle rod (301). A pull-head slot (303) is provided on the circumferential surface of the handle rod (301) along the axial direction of the handle rod (301). The end of the clamping transmission member (6) is connected to the clamping handle (4).

3. The split-type left atrial appendage closure clip system according to Claim 2, **characterized in that** the clamping transmission member (6) is connected to the clamping handle (4) through a pin rod (401) connected to the clamping handle (4).

4. The split-type left atrial appendage closure clip system according to Claim 2, **characterized in that** a step is provided on the inner wall of the central hole of the deflection adjustment sleeve (5). A pull head (701) is provided at the rear end of the deflection transmission member (7). A pair of pull-head pins (704) are radially fixed on the pull head (701). The pull-head pins (704) are threaded through the pull-head slot (303) and extend outside the pull-head slot (303). The part of the pull-head pin (704) exposed outside the pull-head slot (303) is clamped on the step of the inner wall of the central hole of the deflection adjustment sleeve (5).

5. The split-type left atrial appendage closure clip system according to Claim 1, **characterized in that** the clamping transmission member (6) is a cable, which is threaded through the deflection transmission member (7), and the deflection transmission member (7) is threaded inside the handle rod (301).

6. The split-type left atrial appendage closure clip system according to Claim 1, **characterized in that** the clip head comprises a movable clamp arm (1) and a clip head frame (9). A first clamping body (12) is detachably connected to the movable clamp arm (1). A second clamping body (13) is detachably connected to the frame of the clip head frame (9) opposite to the movable clamp arm (1). A clamping and locking structure is provided between the first clamping body (12) and the second clamping body (13). The movable clamp arm (1) is slidably connected inside the clip head frame (9) and connected to the clamping transmission member (6). A rubber band (14) for resetting the movable clamp arm (1) is provided between the movable clamp arm (1) and the clip head frame (9).

7. The split-type left atrial appendage closure clip system according to Claim 6, **characterized in that** the clip head deflection mechanism comprises a connecting lug (901) and a deflection push rod (902). The connecting lug (901) is provided on the clip head frame (9). The connecting lug (901) is hingedly connected to the end of the connecting tube (8). One end of the deflection push rod (902) is hingedly connected to the connecting lug (901), and the other end of the deflection push rod (902) is hingedly connected to the deflection transmission member (7).

8. The split-type left atrial appendage closure clip system according to Claim 6, **characterized in that** a slider guide slot (903) is provided on the frame of the clip head frame (9). A release slider (2) is slidably connected inside the slider guide slot (903). A C-shaped elastic piece (904) is sleeved on the frame of the clip head frame (9). The second clamping body (13) abuts against the inner side of the frame of the clip head frame (9) under the constraint of the C-shaped elastic piece (904). An inclined surface in contact with the C-shaped elastic piece (904) is provided on the back of the release slider (2). An unlocking cable (202) is connected to the release slider (2). The unlocking cable (202) is threaded through the connecting tube (8) and connected to a release handle (10). The release handle (10) is hingedly connected to a release handle ring (11), and the release handle ring (11) is fixed on the fixed handle (3).

9. The split-type left atrial appendage closure clip system according to Claim 6, **characterized in that** the first clamping body (12) and the second clamping body (13) are made of POM.

10. The split-type left atrial appendage closure system according to any one of Claims 1 to 9, **characterized in that** the connecting tube (8) comprises two half-section tubes (801), and the two half-section tubes (801) are joined together.
